(19) Europäisches Patentamt
European Patent Office
Office européen des brevets

(11) **EP 4 432 300 A1**

(12) **EUROPEAN PATENT APPLICATION**

(43) Date of publication:
**18.09.2024 Bulletin 2024/38**

(21) Application number: **23161508.9**

(22) Date of filing: **13.03.2023**

(51) International Patent Classification (IPC):
*G16H 50/20* (2018.01)      *G16H 50/70* (2018.01)
*G16H 40/67* (2018.01)      *G06N 3/045* (2023.01)
*G06N 3/094* (2023.01)      *G06N 3/098* (2023.01)
*G06N 3/0475* (2023.01)      *G06N 3/063* (2023.01)

(52) Cooperative Patent Classification (CPC):
**G06N 3/045; G06N 3/094; G06N 3/098;**
**G16H 40/67; G16H 50/20; G16H 50/70;**
G06N 3/0475; G06N 3/063

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **BALAKRISHNAN, Syamanthaka**
  **Eindhoven (NL)**
• **JAIN, Anshul**
  **Eindhoven (NL)**
• **BUCUR, Anca Ioana Daniela**
  **5656 AG Eindhoven (NL)**
• **IRISARRI MÉNDEZ, Nancy**
  **Eindhoven (NL)**
• **SIDDARTHA, Rachakonda**
  **Eindhoven (NL)**

(74) Representative: **Philips Intellectual Property &**
**Standards**
**High Tech Campus 52**
**5656 AG Eindhoven (NL)**

(54) **METHODS AND SYSTEMS FOR TRAINING MACHINE LEARNING MODELS IN A FEDERATED LEARNING FRAMEWORK**

(57)      The present disclosure is directed to methos and systems of training a machine learning model in a federated learning framework. More specifically, the methods and systems described herein enable broader participation in a federated learning framework by allowing a central coordinating server to perform resource-intensive model training steps for one or more client devices. Further, the methods and systems protect protected information by only sharing primary and secondary inferences of the protected information with the central coordinator system and by creating a synthetic dataset of the protected data at the central coordinator system to train a client-specific model prior to aggregating the model features across multiple client devices.

FIG. 1A

**(Cont. next page)**

EP 4 432 300 A1

100

111 → TRAIN A CLIENT-SPECIFIC ML MODEL FOR A THRESHOLD NUMBER OF TRAINING ITERATIONS

113 → EXTRACT A SET OF PRIMARY AND/OR SECONDARY INFERENCES FROM THE TRAINED CLIENT-SPECIFIC ML MODEL

115 → TRANSMIT THE SET OF PRIMARY AND/OR SECONDARY INFERENCES TO A CENTRAL COORDINATOR SYSTEM

# FIG. 1B

**Description**

FIELD OF THE DISCLOSURE

The present disclosure relates generally to methods and systems for reducing the number of iterations a machine learning model is run in a federated learning framework, and more specifically to methods and systems that employ generative medical information in the operation of a machine learning model within a federated learning framework.

BACKGROUND

When developing machine learning models, a model must be trained using sample data a sufficiently large number of times in order to provide adequate results. Training a machine learning model over a large number of iterations can be prohibitively expensive in terms of time and computational resources. When sample data can be freely shared with other entities for processing, these time and computational costs may not be a problem. However, when handling protected information, such as patient medical data, such information cannot be freely distributed. In some cases, a federated learning framework may be employed to ensure that protected medical data does not leave the premises of the healthcare network. In a federated learning framework, a machine learning model is trained on site at one or more client devices, which then send the model parameters and/or updated model parameters to an off-site central coordinate for aggregation. As a result, each of the client devices are responsible for bearing the computational and time costs associated with running the machine learning model, which often results in large wait times and inefficient or abrupt aggregations in instances where the client devices fail to send back weights to the central coordinator system.

SUMMARY OF THE DISCLOSURE

According to an embodiment of the present disclosure, a method of training a machine learning model in a federated learning framework is provided. The method comprises: (i) establishing a federated learning framework comprising a central coordinator system and one or more client devices in communication with the central coordinator system; at the central coordinator system: (ii) receiving a set of primary inferences from the one or more client devices, wherein the set of primary inferences comprises medical dataset features derived from actual patient information specific to each of the one or more client devices; (iii) generating a set of synthetic medical information for each of the one or more client devices based on the medical dataset features of the received set of primary inferences; (iv) training a plurality of client-specific machine learning models for a minimum number of training iterations, wherein each client-specific machine learning model is trained based on the generated set of synthetic medical information corresponding to that client device; (v) extracting a set of model features from each of the plurality of client-specific machine learning models; (vi) generating a global machine learning model based on the sets of model features extracted from each of the plurality of client-specific machine learning models, wherein the global machine learning model aggregates model features across each of the plurality of client-specific machine learning models; and (vii) transmitting the global machine learning model from the central coordinator system to each of the one or more client devices; and (viii) generating, at the one or more client devices, a prediction using the global machine learning model based on actual patient information specific to each of the one or more client devices.

In an aspect, the method further comprises, at the one or more client devices: training a client-specific machine learning model for a threshold number of training iterations, wherein each client-specific machine learning model is trained based on the actual patient information specific to the corresponding one or more client devices; extracting, from the trained client-specific machine learning model, a set of primary inferences comprising medical dataset features; and transmitting the set of primary inferences to the central coordinator system.

In an aspect, the method further comprises, at the central coordinator system, receiving a set of secondary inferences from the one or more client devices, wherein the set of secondary inferences comprises feature correlations derived from actual patient information specific to each of the one or more client devices, and wherein generating the set of synthetic medical information for each of the one or more client devices is performed based on the medical dataset features of the received set of primary inferences and the feature correlations of the received set of secondary inferences.

In an aspect, the method further comprises, at the one or more client devices: training a client-specific machine learning model for a threshold number of training iterations, wherein each client-specific machine learning model is trained based on the actual patient information specific to the corresponding one or more client devices; extracting, from the trained client-specific machine learning model, a set of primary inferences comprising medical dataset features and a set of secondary inferences comprising feature correlations; and transmitting the set of primary inferences and the set of secondary inferences to the central coordinator system.

In an aspect, the threshold number of training iterations performed at the one or more client devices is less than the minimum number of training iterations performed at the central coordinator system.

In an aspect, the medical dataset features include at least one of a dataset scale, a dataset shape, a dataset threshold, and a dataset distribution.

In an aspect, at least one set of synthetic medical information generated for at least one of the one or more client devices comprises more data points than the actual patient information used to derive the corresponding set of primary inferences.

In an aspect, at least one of the one or more client devices is a limited purpose computer. In an aspect, each of the plurality of client-specific machine learning models and the global machine learning model are neural networks.

In an aspect, a generative adversarial network is used to generate the set of synthetic medical information for each of the one or more client devices based on the medical dataset features of the received set of primary inferences.

In an aspect, the actual patient information specific to each of the one or more client devices is not disclosed to the central coordinator system.

According to another embodiment of the present disclosure, a federated learning framework configured to train a machine learning model is provided. The federated learning framework comprises: a central coordinator system comprising a processor and a set of client devices in communication with the central coordinator system, wherein each client device comprises a processor. The processor of the central coordinator system may be configured to: receive a set of primary inferences from the one or more client devices, wherein the set of primary inferences comprises medical dataset features derived from actual patient information specific to each of the one or more client devices; generate a set of synthetic medical information for each of the one or more client devices based on the medical dataset features of the received set of primary inferences; train a plurality of client-specific machine learning models for a minimum number of training iterations, wherein each client-specific machine learning model is trained based on the generated set of synthetic medical information corresponding to that client device; extract a set of model features from each of the plurality of client-specific machine learning models; generate a global machine learning model based on the sets of model features extracted from each of the plurality of client-specific machine learning models, wherein the global machine learning model aggregates model features across each of the plurality of client-specific machine learning models; and transmit the global machine learning model from the central coordinator system to each of the one or more client devices. Each of the processors of the client devices may be configured to: transmit the set of primary inferences from the client device to the central coordinator system; receive the global machine learning model from the central coordinator system; and generate a prediction using the global machine learning model based on actual patient information specific to the client device.

In an aspect, the processor of each client device is further configured to: train a client-specific machine learning model for a threshold number of training iterations, wherein the client-specific machine learning model is trained based on the actual patient information specific to the client device; and extract, from the trained client-specific machine learning model, a set of primary inferences comprising medical dataset features.

According to still another embodiment of the present disclosure, a central coordinator system adapted to train a machine learning model in a federated learning framework is provided. The central coordinator system comprises a memory in communication with a processor, wherein the processor is configured to: receive a set of primary inferences from one or more client devices, wherein the set of primary inferences comprises medical dataset features derived from actual patient information specific to each of the one or more client devices; generate a set of synthetic medical information for each of the one or more client devices based on the medical dataset features of the received set of primary inferences; train a plurality of client-specific machine learning models for a minimum number of training iterations, wherein each client-specific machine learning model is trained based on the generated set of synthetic medical information corresponding to that client device; extract a set of model features from each of the plurality of client-specific machine learning models; and generate a global machine learning model based on the sets of model features extracted from each of the plurality of client-specific machine learning models, wherein the global machine learning model aggregates model features across each of the plurality of client-specific machine learning models.

In an aspect, the processor of the central coordinator system is further configured to: store the global machine learning model in the memory; and transmit the global machine learning model from the central coordinator system to one or more client devices.

These and other aspects of the various embodiments will be apparent from and elucidated with reference to the embodiments described hereinafter.

BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the various embodiments.

FIG. 1A is a flowchart of a method of training a machine learning model in a federated learning framework illustrated according to aspects of the present disclosure.

FIG. 1B is a flowchart of a method of training a machine learning model in a federated learning framework illustrated according to further aspects of the present disclosure.

FIG. 2 is schematic diagram of a federated learning framework illustrated according to aspects of the present disclosure.

FIG. 3 is a schematic block diagram of a central coordinator system illustrated according to aspects of the present disclosure.

FIG. 4 is a schematic block diagram of a central coordinator system illustrated according to further aspects of the present disclosure.

DETAILED DESCRIPTION OF EMBODIMENTS

The present disclosure is directed to methods and systems that creates synthetic datasets of protected patient information that are used to train a machine learning model within a federated learning framework. As described herein, the methods and systems ensure that protected medical data is not digitally transferred and prevents reverse engineering-based re-identification by duplicating the conceptual data distributions and correlations. The methods and systems further enable participation in a federated learning framework even if the participating client does not have adequate processing power to generate and/or train its own machine learning model.

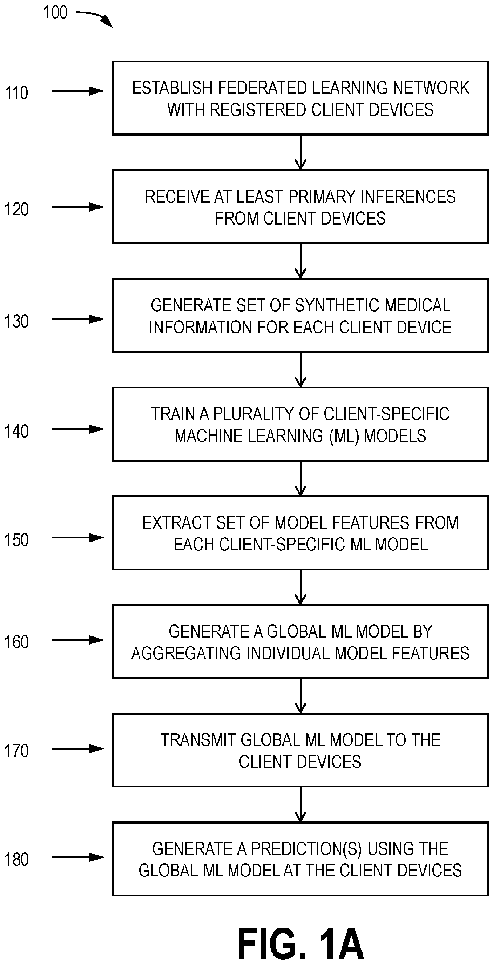

Turning to FIG. 1A, a method 100 of training a machine learning model in a federated learning framework is illustrated according to aspects of the present disclosure.

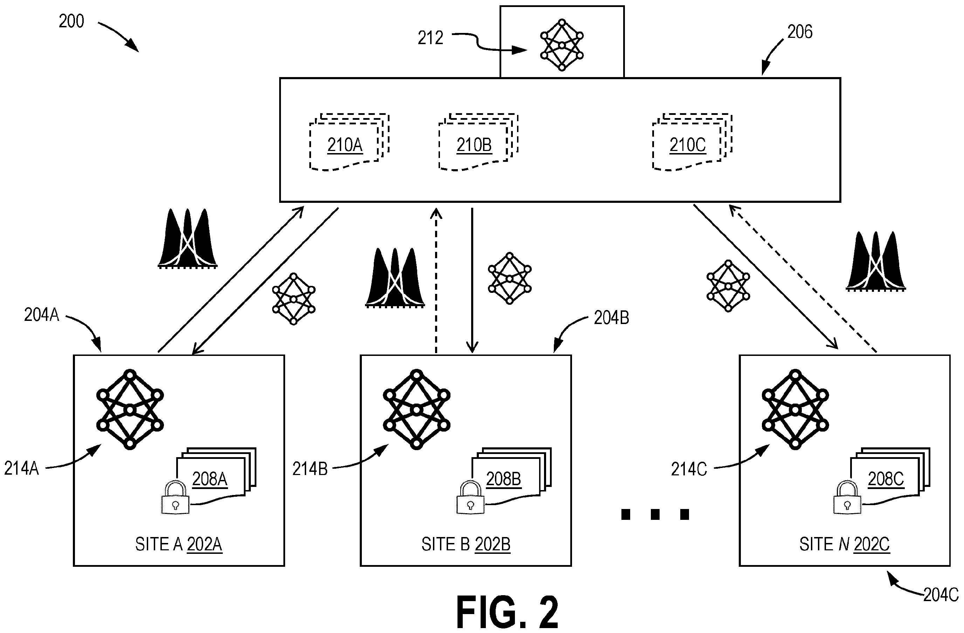

At step 110, the method 100 includes establishing a federated learning network with a central coordinator system and one or more registered client devices. As shown in FIG. 2 and discussed further herein, the federated learning network 200 that is established may span a plurality of different physical sites 202A, 202B, 202C. These sites 202A, 202B, 202C can be, for example and without limitation, one or more different hospitals and/or healthcare clinics, as well as one or more different medical departments (e.g., intensive care units, emergency departments, etc.) within a particular healthcare network.

In embodiments, each of these sites 202A, 202B, 202C may have an electronic device 204A, 204B, 204C with access to protected patient information 208A, 208B, 208C that may not leave the premises of the site (either physically or digitally). As used herein, the terms "client device" refers to these electronic devices 204A, 204B, 204C that are located on the premises of each site 202A, 202B, 202C.

In embodiments, a client device 204A, 204B, 204C may be a special-purpose or general-purpose computer system that includes computer hardware, such as, for example, one or more processors, memory, and/or input/output hardware. In particular embodiments, a client device 204A, 204B, 204C may store or otherwise have access to protected information 208A, 208B, 208C, as discussed herein. In some embodiments, the client device 204A, 204B, 204C may be a "thin client", meaning that the client device 204A, 204B, 204C is a simple, low-performance computer that has minimal processing power and that has been optimized for establishing a remote connection with the central coordinator system 206.

In further embodiments, one or more of the client devices 204A, 204B, 204C may be remote from a central coordinator system 206 that is configured to manage and interface with a plurality of client devices 204A, 204B, 204C. For example, the central coordinator system 206 of the federated learning framework 200 may be a cloud-based server or collection of servers that is not located on the premises of any particular site 202A, 202B, 202C.

In other embodiments, one or more of the client devices 204A, 204B, 204C may be local to the central coordinator system 206. For example, in embodiments, the central coordinator system 206 may be located on the premises of at least one of the sites 202A, 202B, 202C of the one or more client devices 204A, 204B, 204C. As such, in some embodiments, the federated learning network 200 may include a central coordinator system 206 that is local (on-site) to one or more of the client devices 204A, 204B, 204C.

When establishing the federated learning framework 200, the central coordinator system 206 can register each of the one or more client devices 204A, 204B, 204C that will be participating in the federated learning framework 200 and establish a communication link with each of the one or more client devices 204A, 204B, 204C. The central coordinator system 206 may also establish rules regarding the use of the communication link and/or the transfer of information such that any transfer of information is secure (e.g., encrypted, etc.).

At step 120, the central coordinator system 206 receives a set of primary inferences from each of the one or more client devices 204A, 204B, 204C via an established communication link. In embodiments, each set of primary inferences may include medical dataset features derived from actual patient information 208A, 208B, 208C specific to each of the one or more client devices 204A, 204B, 204C. For example, each site 202A, 202B, 202C may be an intensive care unit in a different hospital network and therefore houses different sets of actual patient information 208A, 208B, 208C (e.g., medical records, charts, lab results, diagnoses, etc.) for a plurality of different patients.

Due to various regulatory and data privacy concerns, these sets of actual patient information 208A, 208B, 208C may not be transferred off-site or otherwise shared with a central coordinator system 206. For example, in some embodiments, the protected patient information 208A, 208B, 208C may include physiological data, medical imaging data, diagnoses data, biometric data, and/or the like, including combinations thereof. However, according to the present disclosure, a set of primary inferences that excludes the actual patient information 208A, 208B, 208C can be shared with the central coordinator system 206.

In embodiments, each set of primary inferences includes generalized statistical information and/or metadata corresponding to the actual patient information 208A, 208B, 208C of each client device 204A, 204B, 204C and/or site 202A, 202B, 202C. For example, the primary inferences can include, but is not limited to, at least one of a dataset scale, a dataset shape, a dataset threshold, and a dataset distribution. Accordingly, at step 120, the central coordinator system 206 may receive a plurality of sets of primary inferences from a plurality of client devices 204A, 204B, 204C within the federated learning framework 200 such that each set of primary inferences corresponds to a particular client device 204A, 204B, 204C and/or a particular site 202A, 202B, 202C.

At step 130, the central coordinator system 206 generates a set of synthetic medical information 210A, 210B, 210C for each of the one or more client devices 204A, 204B, 204C based on the corresponding set of primary inferences. That is, for each of the registered client devices 204A, 204B, 204C, the central coordinator system 206 may use the set of primary inferences received from each client device 204A, 204B, 204C to generate a client-specific set of synthetic medical information 210A, 210B, 210C. These sets of synthetic medical information 210A, 210B, 210C exclude the actual patient information 208A, 208B, 208C but mimic the actual patient information 208A, 208B, 208C such that a machine learning model can still be effectively trained without compromising any legal and/or regulatory aspects surrounding the protected patient information 208A, 208B, 208C.

In embodiments, a deep-learning-based generative model may be used to generate each set of synthetic medical information 210A, 210B, 210C based on the features of an underlying medical dataset. For example, the deep-learning-based generative model a generative adversarial network (GAN) model. More particularly, according to the present disclosure, a GAN model can include a deep generative model 'G' and a discriminative model 'D' that engage in a minimax operation. The aim of each deep generative model 'G' is to generate a distribution 'pg' that is similar to the sample data distribution 'pdata' such that the discriminative model 'D' cannot distinguish between the images from the sample data and the model distribution 'pg'.

For example, if x is a real image drawn from the real data distribution 'pdata' and z is a random noise variable, the noise variable z may be transformed into a sample G(z) by a generator network 'G' that synthesizes samples from the distribution 'pg'. The discriminative model D(x) can compute the probability that input data x is from pdata rather than from the generated model distribution 'pg'. In embodiments, D(x) equals zero if x is determined to be from the generated model distribution 'pg', while D(x) equals 1 is x is determined to be from the input data 'pdata'. In embodiments, these GAN models may be trained by solving the following equation:

$$\min_{G} \max_{D} V(D, G) = E_{x \sim p_{data}}[\log D(x)] + E_{z \sim noise}\left[\log\left(1 - D\big(G(z)\big)\right)\right]$$

At step 140, the central coordinator system 206 trains a plurality of client-specific machine learning models based on a training dataset that includes the corresponding set of synthetic medical information 210A, 210B, 210C. That is, for each client device 204A, 204B, 204C, the central coordinator system 206 may take the generated set of synthetic medical information 210A, 210B, 210C and independently train different client-specific machine learning models using at least each corresponding set of synthetic medical information 210A, 210B, 210C. In particular embodiments, one or more training datasets may also include unprotected patient information, including a combination of synthetic medical information 210A, 210B, 210C and unprotected patient information (e.g., information that can be shared without compromising any laws or regulations).

In embodiments, the central coordinator system 206 may train each of the client-specific machine learning models for a minimum number of training iterations (e.g., at least several thousand training iterations, etc.). In embodiments, the number of training iterations performed for each client-specific machine learning model by the central coordinator system 206 is a minimum number of iterations required to obtain a desired fit to the synthetic data (i.e., each set of synthetic medical information 210A, 210B, 210C). As such, by creating a synthetic dataset 210A, 210B, 210C based on the actual patient information 208A, 208B, 208C and performing the required number of iterations on the central coordinator system 206, the federated learning framework 200 is able to free-up resources on the individual client devices 204A, 204B, 204C (which may not have the computational resources necessary to perform the required number of iterations in the first place), without compromising any legal and/or regulatory aspects surrounding the protected patient information 208A, 208B, 208C.

In particular embodiments, one or more of the client-specific machine learning models (as well as the global trained model 212) may be a deep-learning-based model, such as an artificial neural network (ANN) or a simulated neural network (SNN), decision trees, and/or neural-backed decision trees. Each client-specific machine learning model (as well as the global model 212) may be trained and configured to classify the input data (e.g., new patients, new information for existing patients, etc.) and/or make predictions based on the available information. Put another way, the client-specific machine learning models may be trained based on at least the synthetic data 210A, 210B, 210C, aggregated as discussed herein into a global trained model 212, which can then be used at each client site 202A, 202B, 202C to classify and/or make predictions using unprotected patient data without each client site 202A, 202B, 202C needing to train their own model or share protected patient information 210A, 210B, 210C. Although the machine learning models described herein may be a type of neural network, it is contemplated that these machine learning models may include one or more other types of models that can be iteratively trained on actual and/or synthetic sample data, as discussed herein.

At step 150, the central coordinator system 206 may extract from each of the trained client-specific machine learning models a set of model features. In embodiments, each set of model features comprises model weights and/or gradients of the corresponding trained model after at least a minimum number of training iterations.

At step 160, the central coordinator system 206 generates a global trained model 212 based on the sets of model features extracted from the one or more client-specific machine learning models. In embodiments, the global trained model 212 is generated by aggregating the model features (e.g., model weights and/or gradients) from each of the trained client-specific models. In particular embodiments, the central coordinator system 206 may include aggregation logic comprising rules for how the sets of model features are aggregated.

At step 170, the central coordinator system 206 may then transmit the trained global machine learning model 212 to each of the one or more client devices 204A, 204B, 204C. In embodiments, the trained global machine learning model 212 may be transmitted via a wired and/or wireless communication network (e.g., network 314).

In some embodiments, the central coordinator system 206 may evaluate the performance of the global model 212 prior to transmitting the global model 212 to the one or more client devices 204A, 204B, 204C. For example, the performance of the trained global model 212 may be evaluated using the training data, a subset of the training data, or a different dataset comprising actual and/or synthetic medical information. In some embodiments, the performance of the trained global model 212 may be evaluated relative to an existing model, such as an existing client-specific model 214A, 214B, 214C and/or a previous version of the trained global model 212. In embodiments, if the performance of the global model 212 has improved over the performance the existing model, then the central coordinator system 206 may transmit the trained global machine learning model 212 to each of the one or more client devices 204A, 204B, 204C. That is, the central coordinator system 206 may not transmit the trained global machine learning model 212 if the performance of the global model 212 is not better than the existing trained model in use at a particular site 202A, 202B, 202C.

At step 180, at least one of the client devices 204A, 204B, 204C uses the trained global machine learning model 212 received from the central coordinator system 206 in order to analyze additional site-specific protected patient information 208A, 208B, 208C and make predictions based thereon. That is, once the global model 212 is transmitted to the client devices 204A, 204B, 204C, each client device 204A, 204B, 204C may freely use the global model 212 to generate healthcare-related predictions (e.g., likelihood of different outcomes, length of stay, mortality, etc.) using the actual patient information 208A, 208B, 208C specific to each of the one or more client devices 204A, 204B, 204C.

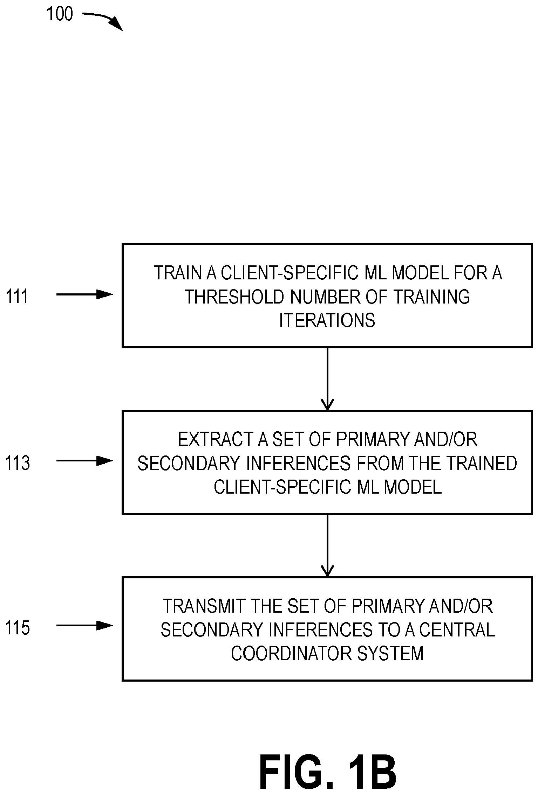

With reference to FIG. 1B, further aspects of a method 100 of training a machine learning model in a federated learning framework are illustrated. In particular, as shown, the method 100 can include: at step 111, training a client-specific machine learning model 214A, 214B, 214C for a threshold number of training iterations using the client device 204A, 204B, 204C; at step 113, extracting a set of primary inferences and/or a set of secondary inferences from the locally-trained models 214A, 214B, 214C; and, at step 115, transmitting the set of primary inferences and/or the set of secondary inferences to the central coordinator system 206.

That is, at step 111, one or more of the client devices 204A, 204B, 204C may perform train a basic machine learning model 214A, 214B, 214C for a threshold number of iterations before the central coordinator system 206 sends back a global trained model 212. In embodiments, the threshold number of iterations may be set such that the client devices 204A, 204B, 204C of the federated learning framework 200 can accomplish the training threshold within a reasonable period of time given the computational resources available to the client devices 204A, 204B, 204C. In some embodiments, the threshold number of training iterations performed by the client devices 204A, 204B, 204C is less than the minimum number of training iterations performed by the central coordinator system 206. In some examples, the minimum number of training iterations performed by the central coordinator system 206 may be 10x greater than the threshold number of training iterations performed by the client devices 204A, 204B, 204C, including at least 100x and at least 1000x greater than the threshold number of training iterations performed by the client devices 204A, 204B, 204C.

Then, at step 113, the client devices 204A, 204B, 204C may extract a set of primary inferences and/or secondary inferences from the minimally-trained client-specific model 214A, 214B, 214C. As discussed above, the primary inferences may include, but is not limited to, a dataset scale, a dataset shape, a dataset threshold, and a dataset distribution.

In embodiments, the set of secondary inferences includes, but is not limited to, feature correlations determined by the minimally-trained client-specific model 214A, 214B, 214C.

Then, at step 115, the client devices 204A, 204B, 204C may transmit the set of primary inferences and/or the set of secondary inferences to the central coordinator system 206 for further training on aggregation into a global model 212. Accordingly, in some embodiments, step 120 may involve receiving one or more sets of secondary inferences comprising feature correlations derived from actual patient information 208A, 208B, 208C.

Although step 110 includes establishing a federated learning framework 200 with a central coordinator system 206 and one or more client devices 204A, 204B, 204C, it should be appreciated that one or more client devices 204A, 204B, 204C may be introduced into the federated learning framework 200 at separate times without being disadvantaged in terms of the global model 212. For example, as discussed below, the central coordinator system 206 may be configured to store the global model 212, update the global model 212 as new information becomes available (e.g., new client devices 204A, 204B, 204C are added, etc.), and/or update each client device 204A, 204B, 204C with the most up-to-date global model 212. Thus, in some embodiments, the method 100 can include storing the global model 212 so that additional and/or newly added client devices 204A, 204B, 204C may receive the global model 212.

With reference to FIG. 2, provided herein are federated learning frameworks 200 configured to train a machine learning model 212. In embodiments, the federated learning framework 200 comprises a central coordinator system 206 and a set of client devices 204A, 204B, 204C in communication with the central coordinator system 206. As described above, each of the one or more client devices 204A, 204B, 204C can be an electronic device with limited computational resources. That is, in embodiments, the one or more client devices 204A, 204B, 204C may be unable to fully train a machine learning model given its limited computational resources.

In specific embodiments, each of the one or more client devices 204A, 204B, 204C has access to protected and/or protected patient information 208A, 208B, 208C, and may comprise one or more processors configured to: (i) transmit a set of primary inferences and/or secondary inferences from the client device 204A, 204B, 204C to the central coordinator system 206; (ii) receive a trained global machine learning model 212 from the central coordinator system 206; and (iii) generate predictions using the trained global machine learning model 212 based on actual patient information 208A, 208B, 208C accessible by the client device 204A, 204B, 204C.

In further embodiments, one or more of the client devices 204A, 204B, 204C may include a processor configured to: (i) train a client-specific machine learning model 214A, 214B, 214C for a limited number of training iterations; and (ii) extract from the trained client-specific machine learning model 214A, 214B, 214C a set of primary inferences and/or a set of secondary inferences. In embodiments, the client-specific machine learning models 214A, 214B, 214C may be minimally trained, as discussed above, but may be trained on the actual patient information 208A, 208B, 208C available to the client device 204A, 204B, 204C.

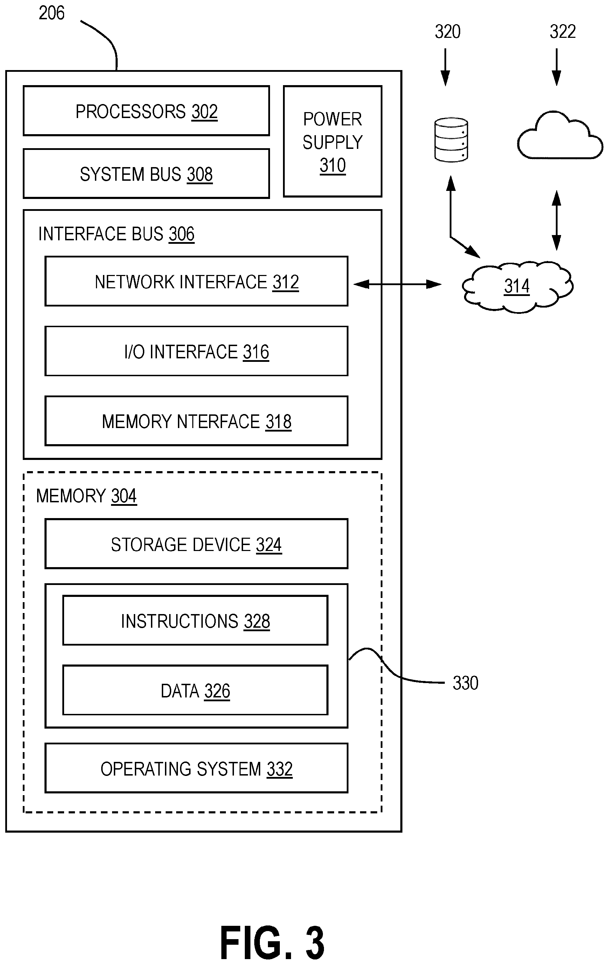

Turning to FIG. 3, a schematic block diagram of a central coordinator system 206 of a federated learning framework 200 is illustrated according to aspects of the present disclosure. In embodiments, the central coordinator system 206 may be embodied as a cloud-based server or collection of servers, or as a server or collection of servers located on one or more of the client sites 202A, 202B, 202C. In further embodiments, the central coordinator system 206 may be adapted train one or more machine learning models in a federated learning framework, as well as manage the entire federated learning workflow by coordinating with individual sites 202A, 202B, 202C and client devices 204A, 204B, 204C.

In the example of FIG. 3, the central coordinator system 206 can include one or more processors 302, machine-readable memory 304, and an interface bus 306, all of which may be interconnected and/or communicate through a system bus 308 containing conductive circuit pathways through which instructions (e.g., machine-readable signals) may travel to effectuate communication, tasks, storage, and the like. The central coordinator system 206 may be connected to a power source 310, which can include an internal power supply and/or an external power supply.

The one or more processors 302 may include a high-speed data processor adequate to execute the program components described herein and/or various specialized processing units as may be known in the art. In some examples, the one or more processors 302 may be a single processor, multiple processors, or multiple processor cores on a single die. In particular embodiments, the one or more processors 302 includes a graphical processing unit (GPU) adequate for training one or more generative adversarial network (GAN) models, one or more neural networks, one or more decision trees, and/or one or more neural-backed decision trees.

In some examples, the interface bus 306 may include a network interface 312 configured to connect the central coordinator system 206 to a communications network 314, an input/output ("I/O") interface 316 configured to connect and communicate with one or more peripheral devices, and/or a memory interface 318 configured to accept, communication, and/or connect to a number of machine-readable memory devices (e.g., memory 304).

The network interface 312 may operatively connect the central coordinator system 206 to a communications network 314, which can include a direct interconnection, the Internet, a local area network ("LAN"), a metropolitan area network ("MAN"), a wide area network ("WAN"), a wired or Ethernet connection, a wireless connection, and similar types of communications networks, including combinations thereof. In some examples, central coordinator system 206 may communicate with one or more remote / cloud-based servers 320, cloud-based services 322, and/or client devices 204A, 204B, 204C via the communications network 314 and the network interface 312.

The memory 304 can be variously embodied in one or more forms of machine-accessible and machine-readable memory. In some examples, the memory 304 includes a storage device 324 comprises one or more types of memory. For example, the storage device 324 can include, but is not limited to, a non-transitory storage medium, a magnetic disk storage, an optical disk storage, an array of storage devices, a solid-state memory device, and the like, including combinations thereof.

Generally, the memory 304 is configured to store data / information 326 and instructions 328 that, when executed by the one or more processors 302, causes the central coordinator system 206 to perform one or more tasks. In particular examples, the memory 304 includes a federated learning package 330 that comprises a collection of program components, database components, and/or data. Depending on the particular implementation, the federated learning package 330 may include software components, hardware components, and/or some combination of both hardware and software components. In some examples, the federated learning package 330 and/or one or more individual software packages may be stored in a local storage device 324. In other examples, the federated learning package 330 and/or one or more individual software packages may be loaded onto and/or updated from a remote server 320 via the communications network 314.

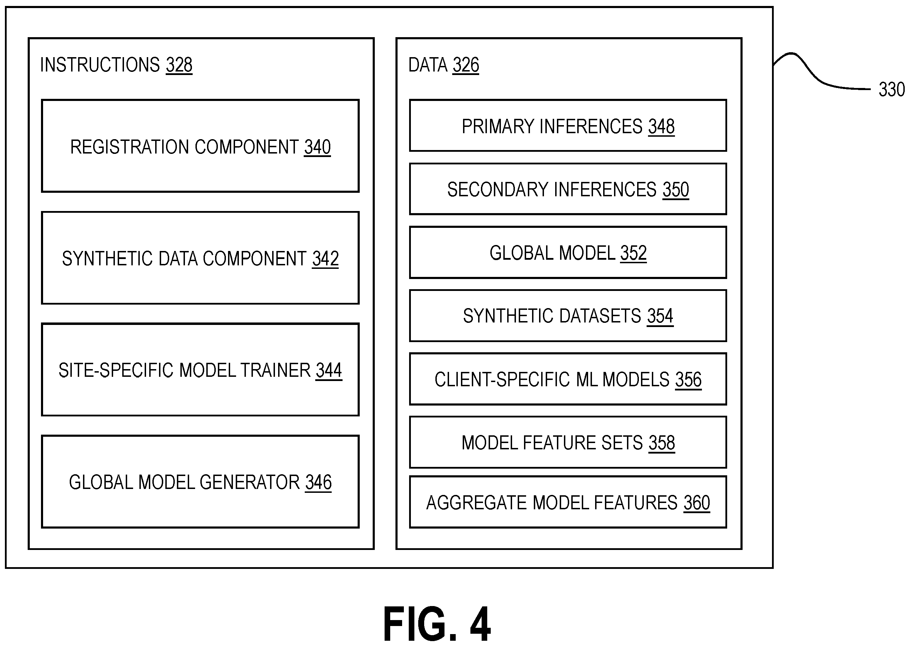

For example, with reference to FIG. 4, the federated learning package 330 may include, but is not limited to, instructions 328 having one or more software packages configured to perform one or more steps of the methods described herein. These software packages may be incorporated into, loaded from, loaded onto, or otherwise operatively available to and from the central coordinator system 206.

In particular embodiments, the federated learning package 330 can include, but is not limited to, instructions 328 having a client device registration component 340, a synthetic data component 342, a model training component 344, and/or a global model component 346. These components may be incorporated into, loaded from, loaded onto, or otherwise operatively available to and from the central coordinator system 206.

The client device registration component 340 can be a stored program component that is executed by at least one processor, such as the one or more processors 302 of the central coordinator system 206. In particular, the client device registration component 340 can be configured to establish a federated learning framework 200 by designating the central coordinator system 206 and registering one or more client devices 204A, 204B, 204C, as described herein. The registration component 340 can be further configured to facilitate data communication between the client devices 204A, 204B, 204C and the central coordinator system 206. For example, in embodiments, the registration component 340 may be configured to facilitate the transfer of different sets of primary inferences 348 and/or secondary inferences 350, which may be stored at data 326 in the central coordinator system 206. In further embodiments, the registration component 340 may be configured to facilitate the transfer of a global model 352 generated by the central coordinator system 206 as described herein.

The synthetic data component 342 can be a stored program component that is executed by at least one processor, such as the one or more processors 302 of the central coordinator system 206. In particular, the synthetic data component 342 can be configured to generate one or more sets of synthetic medical information 354 (e.g., synthetic datasets 210A, 210B, 210C shown in FIG. 2) based on the primary and/or second inferences, 348, 350, as described herein. In particular embodiments, the synthetic data component 342 may include a machine learning model, such as a generative adversarial network (GAN) model that generates a synthetic data 354 of actual medical information 208A, 208B, 208C based on the primary and/or second inferences 348, 350.

The model training component 344 can be a stored program component that is executed by at least one processor, such as the one or more processors 302 of the central coordinator system 206. In particular, the model training component 344 can be configured to train a plurality of client-specific machine learning models 356 based on the synthetic datasets 354 generated by the synthetic data component 342, as described herein. In embodiments, the model training component 344 may train a distinct, machine learning model 356 for each of the one or more client devices 204A, 204B, 204C for which a synthetic dataset 354 was generated. The client-specific machine learning models 356 may be stored as data 326 in the memory 304 of the central coordinator system 206. As described above, each of the client-specific machine learning models 356 can be a neural network, such as an artificial neural network (ANN) or a simulated neural network (SNN), that is trained over at least a minimum number of training iterations.

The global model generation component 346 can be a stored program component that is executed by at least one processor, such as the one or more processors 302 of the central coordinator system 206. In embodiments, the global model generation component 346 can be configured to generate a global machine learning model 352, as described herein. In particular, the global model generation component 346 may extract a set of model features 358 from each of the trained client-specific machine learning models 356. In embodiments, each set of model features 358 can include a plurality of model weights and/or gradients developed by training the individual models 356 on a corresponding synthetic dataset 354. Then, in embodiments, the global model generation component 346 can aggregate the model features 358 into an aggregated set 360 based on certain aggregation logic. Based on the aggregated set of model features 360, the global model generation component 346 may then generate a global model 352.

The central coordinator system 206 may also include an operating system component 332, which may be stored in the memory 304. The operating system component 332 may be an executable program facilitating the operation of the central coordinator system 206. Typically, the operating system component 332 can facilitate access of the I/O interface, network interface, and memory interface, and can communicate with other components of the federated learning network 200.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used herein, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

It should also be understood that, unless clearly indicated to the contrary, in any methods claimed herein that include more than one step or act, the order of the steps or acts of the method is not necessarily limited to the order in which the steps or acts of the method are recited.

The above-described examples of the described subject matter can be implemented in any of numerous ways. For example, some aspects can be implemented using hardware, software or a combination thereof. When any aspect is implemented at least in part in software, the software code can be executed on any suitable processor or collection of processors, whether provided in a single device or computer or distributed among multiple devices/computers.

The present disclosure can be implemented as a system, a method, and/or a computer program product at any possible technical detail level of integration. The computer program product can include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium comprises the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, comprising an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, comprising a local area network (LAN) or a wide area network (WAN), or the connection can be made to an external computer (for example, through the Internet using an Internet Service Provider). In some examples, electronic circuitry comprising, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions can be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions can also be stored in a computer readable storage medium that can direct a computer, a programmable data processing apparatus, and/or other devices to function in a particular manner, such that the computer readable storage medium having instructions stored therein comprises an article of manufacture comprising instructions which implement aspects of the function/act specified in the flowchart and/or block diagram or blocks.

The computer readable program instructions can also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various examples of the present disclosure. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant can be entitled.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A method (100) of training a machine learning model in a federated learning framework, the method comprising:

   establishing (110) a federated learning framework comprising a central coordinator system and one or more client devices in communication with the central coordinator system;
   at the central coordinator system:

   receiving (120) a set of primary inferences from the one or more client devices, wherein the set of primary inferences comprises medical dataset features derived from actual patient information specific to each of the one or more client devices;
   generating (130) a set of synthetic medical information for each of the one or more client devices based on the medical dataset features of the received set of primary inferences;
   training (140) a plurality of client-specific machine learning models for a minimum number of training iterations, wherein each client-specific machine learning model is trained based on the generated set of synthetic medical information corresponding to that client device;
   extracting (150) a set of model features from each of the plurality of client-specific machine learning models;
   generating (160) a global machine learning model based on the sets of model features extracted from each of the plurality of client-specific machine learning models, wherein the global machine learning model aggregates model features across each of the plurality of client-specific machine learning models; and
   transmitting (170) the global machine learning model from the central coordinator system to each of the one or more client devices; and
   generating (180), at the one or more client devices, a prediction using the global machine learning model based on actual patient information specific to each of the one or more client devices.

2. The method (100) of claim 1, further comprising:
   at the one or more client devices:

   training (111) a client-specific machine learning model for a threshold number of training iterations, wherein each client-specific machine learning model is trained based on the actual patient information specific to the corresponding one or more client devices;
   extracting (113), from the trained client-specific machine learning model, a set of primary inferences comprising medical dataset features; and
   transmitting (115) the set of primary inferences to the central coordinator system.

3. The method (100) of claim 1, further comprising:

at the central coordinator system:

receiving (120) a set of secondary inferences from the one or more client devices, wherein the set of secondary inferences comprises feature correlations derived from actual patient information specific to each of the one or more client devices; and
wherein generating (130) the set of synthetic medical information for each of the one or more client devices is performed based on the medical dataset features of the received set of primary inferences and the feature correlations of the received set of secondary inferences.

4. The method (100) of claim 3, further comprising:
at the one or more client devices:

training (111) a client-specific machine learning model for a threshold number of training iterations, wherein each client-specific machine learning model is trained based on the actual patient information specific to the corresponding one or more client devices;
extracting (113), from the trained client-specific machine learning model, a set of primary inferences comprising medical dataset features and a set of secondary inferences comprising feature correlations; and
transmitting (115) the set of primary inferences and the set of secondary inferences to the central coordinator system.

5. The method (100) of claim 4, wherein the threshold number of training iterations performed at the one or more client devices is less than the minimum number of training iterations performed at the central coordinator system.

6. The method (100) of claim 1, wherein the medical dataset features include at least one of a dataset scale, a dataset shape, a dataset threshold, and a dataset distribution.

7. The method (100) of claim 1, wherein at least one set of synthetic medical information generated for at least one of the one or more client devices comprises more data points than the actual patient information used to derive the corresponding set of primary inferences.

8. The method (100) of claim 1, wherein at least one of the one or more client devices is a limited purpose computer.

9. The method (100) of claim 1, wherein each of the plurality of client-specific machine learning models and the global machine learning model are neural networks.

10. The method (100) of claim 1, wherein a generative adversarial network is used to generate the set of synthetic medical information for each of the one or more client devices based on the medical dataset features of the received set of primary inferences.

11. The method (100) of claim 1, wherein the actual patient information specific to each of the one or more client devices is not disclosed to the central coordinator system.

12. A federated learning framework (200) configured to train a machine learning model (212), the federated learning framework (200) comprising:

a central coordinator system (206) comprising a processor (302) configured to:

receive a set of primary inferences (348) from the one or more client devices (204A, 204B, 204C), wherein the set of primary inferences (348) comprises medical dataset features derived from actual patient information (208A, 208B, 208C) specific to each of the one or more client devices (204A, 204B, 204C);
generate a set of synthetic medical information (354) for each of the one or more client devices (204A, 204B, 204C) based on the medical dataset features of the received set of primary inferences (348);
train a plurality of client-specific machine learning models (356) for a minimum number of training iterations, wherein each client-specific machine learning model (356) is trained based on the generated set of synthetic medical information (354) corresponding to that client device (204A, 204B, 204C);
extract a set of model features (358) from each of the plurality of client-specific machine learning models (356);
generate a global machine learning model (212) based on the sets of model features (358) extracted from each of the plurality of client-specific machine learning models, wherein the global machine learning model (212) aggregates model features (358) across each of the plurality of client-specific machine learning models (356); and
transmit the global machine learning model (212) from the central coordinator system (206) to each of the one or more client devices (204A, 204B, 204C); and

a set of client devices (204A, 204B, 204C) in communication with the central coordinator system (206), wherein each client device (204A, 204B, 204C) comprises a processor configured to:

transmit the set of primary inferences (348) from the client device (204A, 204B, 204C) to the central coordinator system (206);
receive the global machine learning model (212) from the central coordinator system (206); and
generate a prediction using the global machine learning model (212) based on actual patient information (208A, 208B, 208C) specific to the client device (204A, 204B, 204C).

13. The federated learning framework (200) of claim 12, wherein the processor of each client device (204A, 204B, 204C) is further configured to:

train a client-specific machine learning model (214A, 214B, 214C) for a threshold number of training iterations, wherein the client-specific machine learning model (214A, 214B, 214C) is trained based on the actual patient information (208A, 208B, 208C) specific to the client device (204A, 204B, 204C); and
extract, from the trained client-specific machine learning model (214A, 214B, 214C), a set of primary inferences (348) comprising medical dataset features.

14. A central coordinator system (206) adapted to train a machine learning model (212) in a federated learning framework (200), the central coordinator system (206) comprising a memory (304) in communication with a processor (302), the processor (302) being configured to:

receive a set of primary inferences (348) from one or more client devices (204A, 204B, 204C), wherein the set of primary inferences (348) comprises medical dataset features derived from actual patient information (208A, 208B, 208C) specific to each of the one or more client devices (204A, 204B, 204C);
generate a set of synthetic medical information (354) for each of the one or more client devices (204A, 204B, 204C) based on the medical dataset features of the received set of primary inferences (354);
train a plurality of client-specific machine learning models (356) for a minimum number of training iterations, wherein each client-specific machine learning model (356) is trained based on the generated set of synthetic medical information (354) corresponding to that client device (204A, 204B, 204C);
extract a set of model features (358) from each of the plurality of client-specific machine learning models (356); and
generate a global machine learning model (212) based on the sets of model features (358) extracted from each of the plurality of client-specific machine learning models (356), wherein the global machine learning model (212) aggregates model features (358) across each of the plurality of client-specific machine learning models (356).

15. The central coordinator system (206) of claim 14, wherein the processor (302) is further configured to:

store the global machine learning model (212) in the memory (302); and
transmit the global machine learning model (212) from the central coordinator system (206) to one or more client devices (204A, 204B, 204C).

100

| 110 | → | ESTABLISH FEDERATED LEARNING NETWORK WITH REGISTERED CLIENT DEVICES |
| 120 | → | RECEIVE AT LEAST PRIMARY INFERENCES FROM CLIENT DEVICES |
| 130 | → | GENERATE SET OF SYNTHETIC MEDICAL INFORMATION FOR EACH CLIENT DEVICE |
| 140 | → | TRAIN A PLURALITY OF CLIENT-SPECIFIC MACHINE LEARNING (ML) MODELS |
| 150 | → | EXTRACT SET OF MODEL FEATURES FROM EACH CLIENT-SPECIFIC ML MODEL |
| 160 | → | GENERATE A GLOBAL ML MODEL BY AGGREGATING INDIVIDUAL MODEL FEATURES |
| 170 | → | TRANSMIT GLOBAL ML MODEL TO THE CLIENT DEVICES |
| 180 | → | GENERATE A PREDICTION(S) USING THE GLOBAL ML MODEL AT THE CLIENT DEVICES |

# FIG. 1A

100 ⟍

111 ⟶
```
TRAIN A CLIENT-SPECIFIC ML MODEL FOR A
THRESHOLD NUMBER OF TRAINING
ITERATIONS
```

113 ⟶
```
EXTRACT A SET OF PRIMARY AND/OR
SECONDARY INFERENCES FROM THE TRAINED
CLIENT-SPECIFIC ML MODEL
```

115 ⟶
```
TRANSMIT THE SET OF PRIMARY AND/OR
SECONDARY INFERENCES TO A CENTRAL
COORDINATOR SYSTEM
```

# FIG. 1B

FIG. 2

**FIG. 3**

EP 4 432 300 A1

INSTRUCTIONS 328

REGISTRATION COMPONENT 340

SYNTHETIC DATA COMPONENT 342

SITE-SPECIFIC MODEL TRAINER 344

GLOBAL MODEL GENERATOR 346

DATA 326

PRIMARY INFERENCES 348

SECONDARY INFERENCES 350

GLOBAL MODEL 352

SYNTHETIC DATASETS 354

CLIENT-SPECIFIC ML MODELS 356

MODEL FEATURE SETS 358

AGGREGATE MODEL FEATURES 360

330

# FIG. 4

Europäisches
Patentamt

European
Patent Office

Office européen
des brevets

# EUROPEAN SEARCH REPORT

Application Number

**EP 23 16 1508**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/051146 A1 (VERMA DINESH C [US] ET AL) 17 February 2022 (2022-02-17) * paragraphs [0003], [0024] – [0026], [0039], [0043]; figures 2,3,5 * ----- | 1-15 | INV.<br>G16H50/20<br>G16H50/70<br>G16H40/67<br>G06N3/045 |
| A | US 2022/237898 A1 (UEHARA DAIKI [JP]) 28 July 2022 (2022-07-28) * the whole document * ----- | 1-15 | G06N3/094<br>G06N3/098<br><br>ADD.<br>G06N3/0475<br>G06N3/063 |

**TECHNICAL FIELDS SEARCHED (IPC)**

G16H
G06N

The present search report has been drawn up for all claims

| Place of search | Date of completion of the search | Examiner |
|---|---|---|
| The Hague | 4 September 2023 | Manfrin, Max |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                

& : member of the same patent family, corresponding document

## ANNEX TO THE EUROPEAN SEARCH REPORT
## ON EUROPEAN PATENT APPLICATION NO.

EP 23 16 1508

This annex lists the patent family members relating to the patent documents cited in the above-mentioned European search report.
The members are as contained in the European Patent Office EDP file on
The European Patent Office is in no way liable for these particulars which are merely given for the purpose of information.

04-09-2023

| Patent document cited in search report | | Publication date | Patent family member(s) | | Publication date |
|---|---|---|---|---|---|
| US 2022051146 | A1 | 17-02-2022 | NONE | | |
| US 2022237898 | A1 | 28-07-2022 | DE 112020005111 | T5 | 03-11-2022 |
| | | | JP 7317136 | B2 | 28-07-2023 |
| | | | JP WO2021079792 | A1 | 29-04-2021 |
| | | | US 2022237898 | A1 | 28-07-2022 |
| | | | WO 2021079792 | A1 | 29-04-2021 |

EPO FORM P0459

For more details about this annex : see Official Journal of the European Patent Office, No. 12/82